# EUROPÄISCHE PATENTANMELDUNG

(11) **EP 4 752 113 A1**
(43) Veröffentlichungstag der Anmeldung: **03.06.2026**
(21) Anmeldenummer: 25200316.5
(22) Anmeldetag: 04.09.2025
(51) Int. Cl.: C02F 1/68, A61K 8/00, A61K 33/20, A61K 33/40, A61L 2/00, A61L 2/18, C02F 1/72, C02F 103/02, C02F 1/461

(54) **ANTIMIKROBIELLE BEHANDLUNG VON PROZESSWASSER**

(30) Priorität: 27.11.2024 DE 102024134992
(71) Anmelder: Dr. Schumacher GmbH, 34323 Malsfeld-Beiseförth (DE)
(72) Erfinder: Schumacher, Sönke, 34323 Malsfeld (DE); Kermanjani, Ali, 34323 Malsfeld (DE); Ballez, Mike, 34323 Malsfeld (DE)
(74) Vertreter: Sandvoß, Stefanie

(57) **Zusammenfassung**

Die Erfindung betrifft ein Verfahren zur antimikrobiellen Behandlung von Prozesswasser bei der Herstellung von Kosmetika, Reinigungsmitteln, Bioziden oder Medizinprodukten, wobei das Verfahren die folgenden Schritte umfasst: a) Zugabe mindestens einer biozid wirkenden Substanz in das Prozesswasser; b) Verwendung des behandelten Prozesswassers in einem Herstellungsprozess für Kosmetika, Reinigungsmittel, Biozide oder Medizinprodukte; und c) Zerfall der mindestens einen biozid wirkenden Substanz; wobei es sich bei der mindestens einen biozid wirkenden Substanz um Wasserstoffperoxid (H₂O₂) handelt.

## Beschreibung

Die Erfindung bezieht sich auf ein Verfahren zur antimikrobiellen Behandlung von Prozesswasser, das bei der Herstellung von Kosmetika, Reinigungsmitteln, Bioziden und Medizinprodukten verwendet wird. Sie bezieht sich ferner auf die Verwendung zersetzbarer Substanzen zur mikrobiellen Kontrolle während der Produktionsprozesse.

Bei der Herstellung von Kosmetika, Reinigungsmitteln und Medizinprodukten ist die Aufrechterhaltung der mikrobiellen Kontrolle während des gesamten Produktionsprozesses entscheidend für die Gewährleistung der Produktsicherheit und -qualität. Traditionell wird dies durch die Verwendung chemischer Konservierungsmittel und antimikrobieller Wirkstoffe erreicht, die direkt zu Produktformulierungen hinzugefügt werden. Diese Zusatzstoffe können jedoch manchmal Hautreizungen verursachen, zur Resistenz von pathogenen Keimen beitragen oder mit Verbraucherpräferenzen für "natürliche" oder "konservierungsmittelfreie" Produkte in Konflikt geraten. Auf der anderen Seite führt die Anfälligkeit für mikrobiologische Verunreinigungen des Prozesswassers dazu, dass Produkten erhöhte Konzentrationen von antimikrobiell aktiven Wirkstoffen zugesetzt werden müssen, damit diese eine lange Haltbarkeit aufweisen.

Die Verwendung von Prozesswasser in der Herstellung bringt potenzielle mikrobielle Kontaminationsrisiken mit sich. Während verschiedene Wasseraufbereitungsmethoden existieren, beinhalten viele dieser Methoden persistente Chemikalien oder komplexe Systeme, die möglicherweise Rückstände im Endprodukt hinterlassen. Insbesondere aber werden den Produkten antimikrobielle Wirkstoffe in höherer Konzentration zugesetzt, um eine Verkeimung zu vermeiden.

Dies kann besonders problematisch für Produkte sein, bei denen Rückstände gesetzlich verboten oder welche für sensible Anwendungen bestimmt sind.

Es wurde erkannt, dass eine Methode zur antimikrobiellen Behandlung von Prozesswasser benötigt wird, die diese Probleme überwindet.

Bekannte Lösungen zur antimikrobiellen Behandlung von mikrobiell anfälligen Produkten in der Herstellung beinhalten oft persistente Chemikalien oder komplexe Systeme.

Die WO 2010/139365 A1 beschreibt eine Formulierung für eine kosmetische Zusammensetzung, welche die Zugabe von zwei antimikrobiellen Wirkstoffen zur kosmetischen Zusammensetzung und das Erzielen einer synergistischen antimikrobiellen Wirkung beinhaltet. Die antimikrobiellen Wirkstoffe können elektrochemisch aktiviertes Wasser und Grapefruitkernextrakt sein.

Ein Nachteil dieser bekannten Lösung ist, dass Rückstände dieser antimikrobiellen Wirkstoffe, z. B. Benzalkoniumchlorid aus dem erwähnten Grapefruitkernextrakt, oder deren Nebenprodukte in den hergestellten Endprodukten verbleiben. Dies kann für Kosmetika, Reinigungsmittel, Biozide und Medizinprodukte problematisch sein und Sicherheitsprobleme in der Anwendung verursachen. Zusätzlich können persistente antimikrobielle Mittel zur Entwicklung von Resistenzen gegenüber diesen Mitteln beitragen.

Es besteht daher weiterhin Bedarf an einer effektiven antimikrobiellen Behandlung für Prozesswasser, die keine Rückstände im Endprodukt hinterlässt, einfach zu implementieren ist und mit den Verbraucherpräferenzen für minimale chemische Zusätze übereinstimmt, während gleichzeitig eine hohe Qualität und Sicherheit des Endprodukts gewährleistet wird. Die Herausforderung besteht darin, eine sofortige mikrobielle Kontrolle während der Herstellung zu gewährleisten, wobei sichergestellt wird, dass keine antimikrobiellen Substanzen dieses Prozesses im Produkt verbleiben, wenn der Kunde dieses verwendet.

Die DE 10 2007 017 502 A1 beschreibt elektrochemisch behandeltes Wasser, das eine umfassende desinfizierende Wirkung gegen Bakterien, Pilze, Viren und Prionen aufweist und für eine Vielzahl von Anwendungen geeignet ist, u.a. im Bereich der Sterilisation medizinischer Geräte. Das Wasser wird erhalten durch Elektrolysieren von Wasser und Verringern der Konzentration der dabei entstandenen Oxidantien.

Die DE 10 2006 008 680 beschreibt ein Verfahren zur Behandlung von Prozesswasser in der Lebensmittelindustrie, bei dem dem Prozesswasser Ozon zugesetzt wird.

Die WO 2012/051728 A1 beschreibt ein Verfahren zur Herstellung von elektroaktiviertem Wasser und dessen Einsatz als Agens zur Entkeimung von Prozesswasser.

Die DE 10 2015 008 960 A1 beschreibt ein Verfahren zur Behandlung von Prozesswasser, in dem dieses mit einem Wirkstoff vermischt wird, der mittels Elektrolyse herstellbar ist.

### Aufgabe der Erfindung

Es ist die Aufgabe der vorliegenden Erfindung, ein alternatives Verfahren zur antimikrobiellen Behandlung von Prozesswasser bei der Herstellung von Kosmetika, Reinigungsmitteln, Bioziden und Medizinprodukten bereitzustellen, das vorzugsweise eine sofortige mikrobielle Kontrolle während der Herstellung gewährleistet und gleichzeitig sicherzustellt, dass keine in diesem Verfahren eingesetzten antimikrobiellen Substanzen im Endprodukt verbleiben, was mit den Verbraucherpräferenzen und regulatorischen Anforderungen für minimale chemische Zusätze übereinstimmt und hohe Qualitäts- und Sicherheitsstandards aufrechterhält.

### Allgemeine Beschreibung der Erfindung

Die Erfindung löst diese Aufgabe mit den Merkmalen der Ansprüche und insbesondere mit einem Verfahren zur antimikrobiellen Behandlung von Prozesswasser bei der Herstellung von Kosmetika, Reinigungsmitteln, Bioziden oder Medizinprodukten, wobei das Verfahren folgende Schritte umfasst:
a. Zugabe mindestens einer biozid wirkenden Substanz in das Prozesswasser als vorbeugende antimikrobielle Behandlung,
b. Verwendung des behandelten Prozesswassers in einem Herstellungsprozess für Kosmetika, Reinigungsmittel, Biozide oder Medizinprodukte, und
c. Zerfall der mindestens einen biozid wirkenden Substanz.

Erfindungsgemäß wird als biozid wirkende Substanz Wasserstoffperoxid eingesetzt.

Im Gegensatz zu der biozid wirkenden Substanz Wasserstoffperoxid, die bei Temperaturen oberhalb von 20 °C und einem Druck von 1013 mbar innerhalb von wenigen Tagen bis Wochen zerfällt, handelt es sich für den Fall, dass das erfindungsgemäße Verfahren ein Verfahren zur antimikrobiellen Behandlung von Prozesswasser bei der Herstellung von Bioziden ist, bei dem Biozid, für dessen Herstellung das behandelte Prozesswasser benötigt bzw. eingesetzt wird, nicht um Wasserstoffperoxid, sondern um ein stabiles Biozid.

Die Zugabe der mindestens einen biozid wirkenden Substanz in das Prozesswasser wird als "vorbeugend" bezeichnet, da durch die vorliegende Erfindung vorgeschlagen wird, dem bei der Herstellung von Kosmetika, Reinigungsmitteln, Bioziden oder Medizinprodukten verwendeten Prozesswasser mit Wasserstoffperoxid standardmäßig mindestens eine biozid wirkende Substanz zuzusetzen, um eine Kontamination der vorgenannten Endprodukte, die oftmals in sensiblen Anwendungen Einsatz finden, ausschließen zu können. Tatsächlich keimbefallenes Prozesswasser wird auf diese Weise antimikrobiell behandelt, und keimfreies Prozesswasser bleibt keimfrei und enthält nach dem Zerfall keine störenden Rückstände der biozid wirkenden Substanz.

Die Verwendung des "behandelten" Prozesswassers in Schritt b. meint eine Verwendung von Prozesswasser, das eine vorhergehende Behandlung gemäß Schritt a. erfahren hat, d.h. behandeltes Prozesswasser ist Prozesswasser, dem mindestens eine biozid wirkende Substanz zugesetzt wurde.

Im Gegensatz zum Stand der Technik bietet das erfindungsgemäße Verfahren eine effektive mikrobielle Kontrolle während des Herstellungsprozesses und stellt gleichzeitig sicher, dass das Endprodukt frei von Rückständen der mindestens einen biozid wirkenden Substanz ist, was den Verbraucherwünschen nach Produkten mit minimalen chemischen Zusätzen entspricht.

Ein Endprodukt ist für die Zwecke der vorliegenden Erfindung "frei von Rückständen" der biozid wirkenden Substanz, wenn <10 ppm der biozid wirkenden Substanz in dem Endprodukt vorhanden ist, was den Grenzwerten der deutschen Trinkwasserverordnung entspricht.

Erfindungsgemäß ist die mindestens eine biozid wirkende Substanz Wasserstoffperoxid (H₂O₂), vorzugsweise in einer Konzentration von 1 - 200 ppm, wobei das Wasserstoffperoxid eine sofortige antimikrobielle Wirkung bereitstellt und die mikrobielle Belastung im Prozesswasser reduziert oder eliminiert und innerhalb weniger Wochen in Wasser und Sauerstoff zerfällt, wodurch sichergestellt wird, dass keine oder nur akzeptable Wasserstoffperoxid-Rückstände gemäß deutscher Trinkwasserverordnung von <10 ppm im Endprodukt verbleiben, wenn es durch den Kunden angewendet wird.

Die Verwendung von Wasserstoffperoxid als biozid wirkende Substanz bietet eine breitspektrale antimikrobielle Wirksamkeit, denn Wasserstoffperoxid zerfällt in harmlose Nebenprodukte, was es zu einer idealen Wahl für die Aufrechterhaltung der Produktsicherheit und -qualität macht.

Optional kann dem Prozesswasser als weitere biozid wirkende Substanz elektrochemisch aktiviertes Wasser (ECA) in einer Konzentration von 1 - 200 ppm zugesetzt werden, wobei das elektrochemisch aktivierte Wasser eine sofortige antimikrobielle Wirkung bereitstellt und die mikrobielle Belastung im Prozesswasser reduziert oder eliminiert; durch den Zerfall des elektrochemisch aktivierten Wassers in Wasser und Chloridionen wird sichergestellt, dass keine oder nur akzeptable Rückstände gemäß deutscher Trinkwasserverordnung von <10 ppm im Endprodukt verbleiben, wenn es durch den Kunden angewendet wird.

Elektrochemisch aktiviertes Wasser bietet eine effektive antimikrobielle Behandlungsoption, die vor Ort erzeugt werden kann, wodurch der Bedarf an Lagerung und Handhabung zusätzlicher Chemikalien reduziert wird.

Vorzugsweise kann ein Gemisch aus den biozid wirkenden Substanzen Wasserstoffperoxid (H₂O₂) und elektrochemisch aktiviertes Wasser (ECA) in einer Gesamtkonzentration von 1 - 200 ppm verwendet werden, wobei beide Substanzen eine sofortige antimikrobielle Wirkung bereitstellen und die mikrobielle Belastung im Prozesswasser reduzieren oder eliminieren. Durch den Zerfall des elektrochemisch aktivierten Wassers und des Wasserstoffperoxids (H₂O₂) in Wasser, Sauerstoff und Chloridionen wird sichergestellt, dass keine oder nur akzeptable Rückstände gemäß deutscher Trinkwasserverordnung von <10 ppm im Endprodukt verbleiben, wenn es durch den Kunden angewendet wird.

Die Mischung dieser bioziden Substanzen bietet eine breite und effektive antimikrobielle Behandlungsoption des Prozesswassers.

Unabhängig davon, ob in dem erfindungsgemäßen Verfahren nur eine biozid wirkende Substanz oder mehrere biozid wirkende Substanzen in das Prozesswasser gegeben wird/werden, beträgt die Gesamtkonzentration der in das Prozesswasser gegebenen biozid wirkenden Substanz(en) vorzugsweise 1 bis 200 Teile pro Million (ppm) und besonders bevorzugt 1 bis 50 Teile pro Million (ppm). Dieser Konzentrationsbereich gewährleistet eine effektive mikrobielle Kontrolle bei gleichzeitiger Minimierung des Potenzials für nachteilige Auswirkungen auf die Produktformulierung oder den Herstellungsprozess.

Das behandelte Prozesswasser kann in einem Formulierungsschritt verwendet werden, in dem das behandelte Prozesswasser als Basis für die Produktion von Kosmetika, Reinigungsmitteln, Bioziden oder Medizinprodukten verwendet wird.

Das erfindungsgemäße Verfahren trägt durch die Bereitstellung des antimikrobiell behandelten Prozesswassers dazu bei, mikrobielle Kontamination an einem kritischen Punkt im Herstellungsprozess von Kosmetika, Reinigungsmitteln, Bioziden oder Medizinprodukten zu verhindern und erhöht die allgemeine Produktsicherheit. Zudem führt das erfindungsgemäße Verfahren dazu, dass die Konzentration von Konservierungsmitteln in den Endprodukten reduziert werden kann, ohne dass die Sicherheit dieser eingeschränkt wird.

Gemäß einer Ausführungsform kann das in dem erfindungsgemäßen Verfahren eingesetzte Prozesswasser entionisiertes Wasser sein.

Die Verwendung von entionisiertem Wasser als Prozesswasser reduziert das Potenzial für Störungen durch Mineralien oder andere Verunreinigungen und gewährleistet eine konsistente und vorhersehbare antimikrobielle Wirksamkeit.

Alternativ kann das in dem erfindungsgemäßen Verfahren eingesetzte Prozesswasser auch teilentionisiertes Wasser oder unbehandeltes Leitungswasser sein.

Das erfindungsgemäße Verfahren kann weiter die Überwachung der Konzentration der biozid wirkenden Substanz(en) im Prozesswasser während des Herstellungsprozesses für Kosmetika, Reinigungsmittel, Biozide oder Medizinprodukte umfassen.

Die kontinuierliche Überwachung der Konzentration(en) der biozid wirkenden Substanz(en) ermöglicht Echtzeitanpassungen zur Aufrechterhaltung einer optimalen antimikrobiellen Wirksamkeit während des gesamten Herstellungsprozesses.

Dabei kann die Überwachung die Verwendung eines oder mehrerer Schnelltestkits oder eines oder mehrerer Sensorgeräts/Sensorgeräte zur Erkennung der Anwesenheit der biozid wirkenden Substanz(en) umfassen.

Die Verwendung von Schnelltestkits oder Sensorgeräten ermöglicht eine schnelle und genaue Überwachung der Konzentration der mindestens einen biozid wirkenden Substanz und erleichtert eine effiziente Prozesskontrolle und Qualitätssicherung.

Die biozid wirkende Substanz kann unter typischen Herstellungs- und Lagerbedingungen (20 °C, 1013 mbar) innerhalb mehrerer Wochen zerfallen.

Dieser Zerfallszeitraum stellt sicher, dass die Anwesenheit der biozid wirkenden Substanz während der kritischen Herstellungsphasen in einem Herstellungsprozess für Kosmetika, Reinigungsmittel, Biozide oder Medizinprodukte Schutz bietet, während ihre Abwesenheit im Endprodukt garantiert wird, was mit den Verbraucherpräferenzen für Produkte ohne unnötige chemische Zusätze übereinstimmt.

### Genaue Beschreibung der Erfindung

Die Erfindung wird nun anhand von Ausführungsbeispielen und unter Bezugnahme auf die Abbildungen näher beschrieben. Diese zeigen:
- in der FIG. 1 ein Diagramm, das den Zerfall von Wasserstoffperoxid (H₂O₂) über die Zeit bei 25°C darstellt;
- in der FIG. 2 ein Diagramm, das den Zerfall von Wasserstoffperoxid (H₂O₂) über die Zeit bei 40°C darstellt.

Die vorliegende Offenbarung bezieht sich auf ein Verfahren zur antimikrobiellen Behandlung von Prozesswasser, das bei der Herstellung von Kosmetika, Reinigungsmitteln, Bioziden und Medizinprodukten verwendet wird. Genauer gesagt, stellt die Offenbarung ein Verfahren unter Verwendung zersetzbarer Substanzen in Prozesswasser bereit, um eine mikrobielle Kontrolle während der Produktion von Kosmetika, Reinigungsmitteln, Bioziden oder Medizinprodukten zu erreichen und gleichzeitig die Abwesenheit von zusätzlichen Rückständen antimikrobieller Wirkstoffe in den vorgenannten Endprodukten sicherzustellen.

In verschiedenen Aspekten beinhalten das Verfahren die Zugabe einer oder mehrerer biozid wirkender Substanzen zum Prozesswasser als vorbeugende antimikrobielle Behandlung. Diese Substanzen werden ausgewählt, um eine sofortige antimikrobielle Wirkung zu erzielen und die mikrobielle Belastung im Prozesswasser zu reduzieren oder zu eliminieren. Das behandelte Prozesswasser wird dann in Herstellungsprozessen für Kosmetika, Reinigungsmittel, Biozide oder Medizinprodukte verwendet.

Ein Hauptmerkmal des offenbarten Verfahrens ist die Verwendung von biozid wirkenden Substanzen, die mit der Zeit zerfallen. Dieser Zerfall stellt sicher, dass keine Rückstände biozider Wirkstoffe im Endprodukt verbleiben, was den Verbraucherpräferenzen für minimale chemische Zusätze entspricht und gleichzeitig hohe Qualitäts- und Sicherheitsstandards aufrechterhält.

Zu den für die antimikrobielle Behandlung verwendeten Substanzen gehören unter anderem Wasserstoffperoxid (H₂O₂) und/oder elektrochemisch aktiviertes Wasser. Diese Substanzen bieten eine breitspektrale antimikrobielle Wirksamkeit und zerfallen in harmlose Nebenprodukte wie Wasser, Sauerstoff und Chlorid-Ionen.

Die hier beschriebenen Verfahren sind auf verschiedene Stufen von Herstellungsprozessen anwendbar, einschließlich Formulierungsschritte. Die Konzentration der biozid wirkenden Substanz(en) im Prozesswasser liegt vorzugsweise im Bereich von 1 bis 200 Teilen pro Million (ppm), wobei je nach spezifischen Herstellungsanforderungen auch andere Konzentrationen möglich sind.

In einigen Aspekten umfasst das Verfahren die Überwachung der Konzentration der biozid wirkenden Substanz im Prozesswasser während der Herstellung. Diese Überwachung erfolgt insbesondere durch die Verwendung von Schnelltestkits oder Sensorgeräten, die Echtzeitanpassungen ermöglichen, um eine optimale antimikrobielle Wirksamkeit aufrechtzuerhalten.

Das offenbarte Verfahren bietet eine effektive mikrobielle Kontrolle während kritischer Herstellungsphasen und adressiert gleichzeitig die Herausforderungen, die mit traditionellen Konservierungsmitteln und antimikrobiellen Wirkstoffen verbunden sind, bei deren Verwendung permanent sichergestellt werden muss, dass die Dosierung des Konservierungsmittels ausreichend hoch ist, um eine Verkeimung der Produkte zu vermeiden.

### Beispiel 1: Herstellung kosmetischer getränkter Tücher unter Anwendung des erfindungsgemäßen Verfahrens

In einer Ausführungsform des Verfahrens wird Wasserstoffperoxid als biozid wirkende Substanz zur antimikrobiellen Behandlung von Prozesswasser verwendet. Die anfängliche Konzentration von H₂O₂ beträgt etwa 20 ppm. Das behandelte Prozesswasser wird dann dazu genutzt, um getränkte Tücher für den kosmetischen Bereich herzustellen, die weiterhin neben dem Prozesswasser 0,1 % Benzoesäure in dem Tränkungsmittel enthalten. Der verwendete Vliesstoff ist zu 100 % natürlichen Ursprungs.

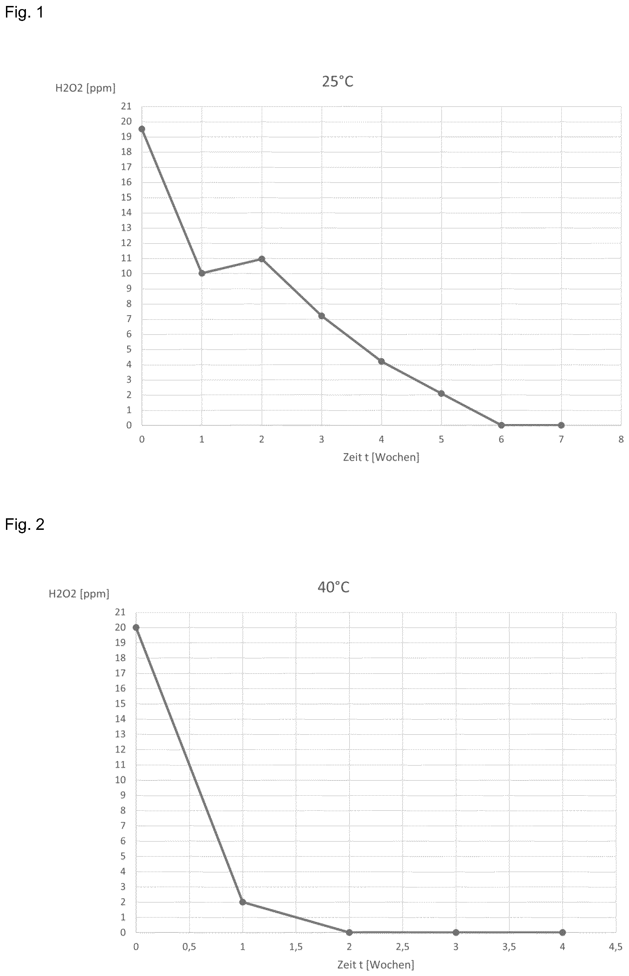

Unter Bezugnahme auf FIG. 1 zeigt das Diagramm den Zerfall von Wasserstoffperoxid (H₂O₂) über die Zeit bei 25°C. Das Diagramm stellt die Konzentration von H₂O₂ in Teilen pro Million (ppm) auf der y-Achse und die Zeit in Wochen auf der x-Achse dar.

FIG. 1 zeigt einen schnellen Rückgang der H₂O₂-Konzentration auf etwa 10 ppm während der ersten Woche. Diese Konzentration wäre bereits im Einklang mit der deutschen Trinkwasserverordnung. Auf diesen anfänglichen schnellen Zerfall folgt eine Periode langsameren Zerfalls.

Die Zerfallsrate von H₂O, bei 25°C wird über einen Zeitraum von 8 Wochen beobachtet. Der vollständige Zerfall, bei dem die H₂O₂-Konzentration 0 ppm erreicht, tritt bis Woche 6 ein. Die Konzentration bleibt bis zum Ende des Beobachtungszeitraumes bei 0 ppm, was darauf hinweist, dass nach dieser Zeit kein H₂O₂ mehr im Prozesswasser vorhanden ist.

Die Zerfallsrate von H₂O₂ im Prozesswasser wird durch Faktoren wie Temperatur, pH-Wert und das Vorhandensein katalytischer Verunreinigungen beeinflusst. Das Diagramm in FIG. 1 stellt den Zerfall bei 25°C dar, was innerhalb des typischen Temperaturbereichs für die Lagerung von Endprodukten liegt.

Das beobachtete Zerfallsprofil deutet darauf hin, dass H₂O₂ bei Zugabe zum Prozesswasser eine sofortige antimikrobielle Wirkung erzielt und diese Wirkung über mehrere Wochen fortsetzt. Dieser verlängerte Wirkungszeitraum gewährleistet eine mikrobielle Kontrolle während des gesamten Herstellungsprozesses und ermöglicht gleichzeitig ausreichend Zeit für einen vollständigen Zerfall, bevor das Produkt den Verbraucher erreicht.

Unter Bezugnahme auf FIG. 2 zeigt ein Diagramm den Zerfall von Wasserstoffperoxid (H₂O₂) über die Zeit bei 40°C. Das Diagramm stellt die Konzentration von H₂O₂ in Teilen pro Million (ppm) auf der y-Achse und die Zeit in Wochen auf der x-Achse dar.

Die anfängliche Konzentration von H₂O₂ beträgt etwa 20 ppm. Das Diagramm zeigt einen schnellen Rückgang der H₂O₂-Konzentration während der ersten Woche auf etwa 2 ppm. Auf diesen anfänglichen schnellen Zerfall folgt ein weiterer Rückgang, wobei die Konzentration bis Woche 2 auf 0 ppm sinkt.

Die Zerfallsrate von H₂O₂ ist bei 40°C deutlich schneller im Vergleich zur Zerfallsrate bei 25°C, die in FIG. 1 gezeigt ist. Der vollständige Zerfall von H₂O₂ erfolgt bei 40°C innerhalb von 2 Wochen, während er bei 25°C etwa 6 Wochen dauert.

Erfindungsgemäß zerfällt die biozid wirkende Substanz innerhalb mehrerer Wochen, z.B. innerhalb von 1-6 Wochen durch natürlichen Zerfall. Die Zerfallszeit variiert dabei in Abhängigkeit von Faktoren wie Temperatur, pH-Wert und dem Vorhandensein katalytischer Verunreinigungen.

Dieser schnelle Zerfall ist vorteilhaft bei Herstellungsprozessen mit kürzeren Produktionszyklen oder höheren Betriebstemperaturen, da er die vollständige Eliminierung der biozid wirkenden Substanz vor Erreichen des Verbrauchers sicherstellt.

Die Konzentration von H₂O₂ im Prozesswasser kann optional mit Hilfe von Schnelltestkits gemessen werden, wie z.B. von Sigma oder Quantofix hergestellt. Diese Kits liefern schnelle und genaue Messungen, die es den Bedienern ermöglichen, die gewünschte Konzentration während des gesamten Herstellungsprozesses zu überwachen und aufrechtzuerhalten. Alternativ werden Sensorgeräte zur kontinuierlichen Überwachung der Substanzkonzentration eingesetzt. Diese Sensoren liefern Echtzeitdaten über die antimikrobiellen Konzentrationen im Prozesswasser und ermöglichen automatisierte Anpassungen zur Aufrechterhaltung der gewünschten Konzentration.

In einer weiteren Ausführungsform des Verfahrens wird eine Kombination aus H₂O₂ und ECA-Wasser als biozid wirkende Substanz zur antimikrobiellen Behandlung von Prozesswasser verwendet.

Das behandelte Prozesswasser, das H₂O₂ und elektrochemisch aktiviertes Wasser jeweils in einer Konzentration von < 10 ppm, also in einer Gesamtkonzentration von < 20 ppm enthält, wird dazu genutzt, um getränkte Tücher für den kosmetischen Bereich herzustellen, die weiterhin neben dem Prozesswasser 0,1 % Benzoesäure in dem Tränkungsmittel enthalten. Der verwendete Vliesstoff ist zu 100 % natürlichen Ursprungs.

Im Laufe der Zeit zerfallen H₂O₂ und das elektrochemisch aktivierte Wasser in Wasser, Sauerstoff und Chloridionen. Dieser Zerfallsprozess stellt sicher, dass keine bioziden Wirkstoffe im Endprodukt verbleiben. Die Zerfallsrate von elektrochemisch aktiviertem Wasser hängt von Faktoren wie Temperatur, pH-Wert und dem Vorhandensein organischer Substanzen ab.

Die in der obigen Beschreibung, in den Abbildungen und in den Ansprüchen offenbarten Merkmale der Erfindung können sowohl einzeln als auch in jeder Kombination für die Umsetzung der Erfindung in ihren verschiedenen Ausführungsformen wesentlich sein.

## Patentansprüche

1. Verfahren zur antimikrobiellen Behandlung von Prozesswasser bei der Herstellung von Kosmetika, Reinigungsmitteln, Bioziden oder Medizinprodukten, **dadurch gekennzeichnet, dass** das Verfahren die folgenden Schritte umfasst:
a) Zugabe mindestens einer biozid wirkenden Substanz in das Prozesswasser;
b) Verwendung des behandelten Prozesswassers in einem Herstellungsprozess für Kosmetika, Reinigungsmittel, Biozide oder Medizinprodukte; und
c) Zerfall der mindestens einen biozid wirkenden Substanz;
wobei es sich bei der mindestens einen biozid wirkenden Substanz um Wasserstoffperoxid (H₂O₂) handelt.

2. Verfahren nach Anspruch 1, **dadurch gekennzeichnet, dass** elektrochemisch aktiviertes (ECA) Wasser als weitere biozid wirkende Substanz in das Prozesswasser zugegeben wird.

3. Verfahren nach einem der voranstehenden Ansprüche, wobei die mindestens eine biozid wirkende Substanz in einer Gesamtkonzentration von 1 bis 200 Teilen pro Million (ppm) in das Prozesswasser zugegeben wird.

4. Verfahren nach einem der voranstehenden Ansprüche, wobei die mindestens eine biozid wirkende Substanz in einer Gesamtkonzentration von 1 bis 50 Teilen pro Million (ppm) zugegeben wird.

5. Verfahren nach einem der voranstehenden Ansprüche, wobei das Prozesswasser entionisiertes Wasser ist.

6. Verfahren nach einem der voranstehenden Ansprüche, weiter umfassend die Überwachung der Konzentration der Substanz im Prozesswasser während des Herstellungsprozesses.

7. Verfahren nach Anspruch 6, wobei die Überwachung die Verwendung eines Schnelltestkits oder eines Sensorgeräts zur Erkennung der Anwesenheit der Substanz umfasst.

8. Verfahren nach einem der Ansprüche 7 oder 8, **dadurch gekennzeichnet, dass** während der Überwachung der Konzentration der Substanz in dem Prozesswasser eine Anpassung der Konzentration der Substanz erfolgt, dadurch dass eine zusätzliche Menge der mindestens einen biozid wirkenden Substanz in das Prozesswasser gegeben wird, wenn festgestellt wird, dass die Konzentration der mindestens einen biozid wirkenden Substanz in dem Prozesswasser einen vorbestimmten Grenzwert unterschreitet.

9. Verfahren nach einem der voranstehenden Ansprüche, wobei die in das Prozesswasser zugegebene mindestens eine biozid wirkende Substanz in dem Herstellungsprozess für Kosmetika, Reinigungsmittel, Biozide oder Medizinprodukte innerhalb von max. 6 Wochen zerfällt.

10. Verfahren nach Anspruch 9, wobei die in das Prozesswasser zugegebene mindestens eine biozid wirkende Substanz in dem Herstellungsprozess für Kosmetika, Reinigungsmittel, Biozide oder Medizinprodukte bei 20 °C und einem Druck von 1013 mbar innerhalb von max. 6 Wochen in Wasser und Sauerstoff zerfällt.
